# EUROPEAN PATENT SPECIFICATION

(11) **EP 1 745 121 B1**
(45) Date of publication and mention of the grant of the patent: **03.04.2019**
(21) Application number: 05735750.1
(22) Date of filing: 04.05.2005
(51) Int. Cl.: C12M 3/00

(54) **BIOREACTOR FOR TISSUE ENGINEERING**
BIOREAKTOR ZUM GEWEBEAUFBAU
BIOREACTEUR POUR GENIE TISSULAIRE

(30) Priority: 06.05.2004 US 568255 P
(43) Date of publication of application: 24.01.2007
(73) Proprietor: University Hospital of Basel, 4031 Basel (CH)
(72) Inventor: JAKOB, Marcel, CH-8586 Andwil (CH); JAKOB, Karl, CH-8586 Andwil (CH); MARTIN, Ivan, CH-4104 Oberwil (CH); TIMMINS, Nicholas, Eion, CH-4056 Basel (CH)
(74) Representative: Latscha Schöllhorn Partner AG
(86) International application number: PCT/IB2005/001210
(87) International publication number: WO 2005/108550

(56) References cited:
- WO-A-01/21760
- US-A- 5 071 766
- US-A1- 2003 143 727

## Description

### BACKGROUND OF THE INVENTION

### 1. Field of the invention

The present invention is a bioreactor for seeding and culturing cells in a three-dimensional environment.

### 2. Background Art

There is increasing interest in studying cell function not in two-dimensional (2D) cultures (e.g., monolayers), but in three-dimensional (3D) environments (e.g., those provided by porous scaffolds). Cell culture in 3D scaffolds can also be useful in cellular therapy for the expansion of functional cells, and tissue engineering for the generation of implantable tissue structures. Intrinsic difficulties with 3D cultures in 3D scaffolds are (i) the uniform and efficient seeding of cells throughout the scaffold pores, and (ii) limited mass transfer to the cells in the central scaffold part. The present inventors have recently reported that perfusion of cell suspensions, and subsequently culture medium through the scaffold pores, is a possible solution for both issues (Wendt et al., Biotechnol. Bioeng. 84:215-214, 2003). WO 01/21760 A2 describes a method of in vitro culturing cells. Thereby cells are introduced into a three-dimensional matrix which is moved in a liquid cell culture medium. US 2003/0143727 A1 describes a method and a device for in vitro cultivating cells with minimal mortality and for harvesting cellular products produced thereof, by way of plating cells and causing maximum adherence of cells of interest. US 5,071,766 A describes an apparatus for isolating and culturing microorganisms which maintains a biological sample on a support in contact with a liquid medium at all times, regardless of the orientation of the apparatus.

The present invention relates to the development of a bioreactor system, where the movement of a holder containing [zl] porous scaffolds generates perfusion of a cell suspension or culture medium through the same scaffolds in alternate directions. The holder could be fully made of porous scaffold material, thus maximizing utilization of the surface. In parallel, or prior to moving the holder, inside the vessel a stirring bar can be moved, so that cells can be cultured in suspension in the vessel with or without mixing.

### SUMMARY OF THE INVENTION

This invention relates to a bioreactor for tissue engineering and a method of operating such a bioreactor.

A first preferred embodiment of the invention is a method for growing cells in culture, comprising the steps of providing a three-dimensional porous scaffold disposed in a culture chamber, the culture chamber comprising a space between a first end cap and a second end cap; providing one or more living cells; providing a liquid growth medium; and causing relative motion between the scaffold and at least one end cap, thereby moving the growth medium through the scaffold. The method of the first preferred embodiment is characterized in that a holder configured to hold the porous, three-dimensional scaffold for growing or maintaining cells is integral with the one said end cap, a hollow rod is, at one end, attached to the center of said one of said end caps and, at the other end, to a punch serving as said holder and fitting an interior diameter of the culture chamber, and, during the step of causing relative motion between the scaffold and at least one end cap, said scaffold moves in conjunction with the one said end cap.

In a further preferred embodiment, the scaffold of the first embodiment may alternately be disposed in one of (1) an upper position out of said growth medium, or (2) a lower position in said growth medium; the method further comprising the steps, before the step of causing relative motion, of: growing said cells in a suspension in said growth medium while said scaffold is disposed in the upper position, and lowering said scaffold into the lower position.

In still another preferred embodiment, the culture chamber of the first embodiment further comprises connectors for flowing said growth medium in and out of said culture chamber.

In yet another preferred embodiment, the culture chamber of the first embodiment further comprises a stirrer bar.

Still another preferred embodiment includes a step, before the step of causing relative motion, of growing said cells in a suspension in said growth medium.

The second preferred embodiment of the invention is a bioreactor for growing cells in culture, comprising a culture chamber including a first end cap and a second end cap connected to define a space, and a holder configured to hold a porous, three-dimensional scaffold for growing or maintaining cells, wherein the holder is disposed between the end caps and is arranged so a reciprocating motion may occur between the holder and at least one of said end caps. The bioreactor according to the second preferred embodiment is characterized in that said holder is integral with one of said end caps and a hollow rod is, at one end, attached to the center of said one of said end caps and, at the other end, to a punch serving as said holder and fitting an interior diameter of the culture chamber.

In yet another preferred embodiment, the holder in the bioreactor of the second preferred embodiment moves in conjunction with one end cap.

In still another preferred embodiment, the bioreactor of the second preferred embodiment further comprises a drive means operationally connected to actuate the reciprocating motion.

In still another preferred embodiment, when a porous scaffold is housed in said chamber for cell culture of the second embodiment, the scaffold may alternately be disposed in one of (1) an upper position out of a growth medium, or (2) a lower position in a growth medium.

In still another preferred embodiment, the culture chamber of the second embodiment further comprises connections disposed to permit flow of a growth medium in and out of said culture chamber.

In yet another preferred embodiment, the second embodiment further comprises a gas port connected to the culture chamber.

In still another preferred embodiment, the second embodiment further comprises a porous, three-dimensional scaffold for growing cells.

Still another preferred embodiment is any of the above-mentioned methods wherein the scaffold comprises a tissue (wherein tissue refers to both living tissues and devitalized/decellularized tissue, and equivalents).

Yet another preferred embodiment is any of the above-mentioned bioreactors wherein said scaffold comprises a tissue.

Yet another preferred embodiment is the first-mentioned method embodiment, wherein the last step or a step following the last step comprises unidirectional perfusion of said medium through said scaffold.

Still another preferred embodiment is the bioreactor of the second embodiment, further comprising two ports for a growth media disposed so that, when the bioreactor houses a scaffold, the growth media is flowable through the ports and said scaffold in a unidirectional manner

### BRIEF DESCRIPTION OF THE DRAWINGS

**Figure 1** shows a bottom unit, or end cap, of the present invention.
**Figure 2** show a top unit, or end cap, of the present invention: Fig. 2A shows the top unit with a scaffold holder and Fig. 2B shows the unit without a scaffold holder.
**Figure 3** shows an assembled bioreactor of the present invention.
**Figure 4** shows a bioreactor of the present invention. Fig. 4A shows the bioreactor standing alone, and Fig. 4B shows a linear drive unit, and Fig. 4C shows the bioreactor mounted to the drive unit.

### DETAILED DESCRIPTION OF THE INVENTION

Advantages of the present invention over existing perfusion bioreactors for cell culture include: (i) simple but highly flexible mode of operation, (ii) possibility of integrating the phases of suspension culture with subsequent perfusion through a scaffold in a closed system, and (iii) easy scale-up.

The present invention may be used for: (i) 'easy-to-use' bioreactors to support research of cell function in a 3D environment; (ii) bioreactors for the culture of cells in a 3D environment for an efficient expansion (e.g., hematopoietic progenitor cells) or for the specialized production of specific proteins (e.g., antibodies, enzymes, agonists, antagonists, hormones, drugs); (iii) bioreactors for the automated generation of 3D tissues for clinical implantation or extracorporeal life support (e.g., bioartificial liver assist device) ; and (iv) bioreactors for the maintenance or revitalization of tissue.

One advantage of 3D cell culture is that certain cells, for example stem cells, may exhibit enhanced properties (e.g., greater retention of progenitor traits) when grown in 3D as compared to conventional growth in a monolayer.

The bioreactor consists of one chamber (optionally containing a stirring bar where cells can be cultured in suspension), capable of holding one or more porous scaffolds which - by reciprocal movement relative to at least one end of the chamber - generates the perfusion of a cell suspension or culture medium through the scaffold pores. Although the scaffold holder, or punch, is described in the examples as a separate component, in some instances the plunger itself may be the scaffold. Neither the scaffold nor holder is limited to any particular shape. The scaffold may consist of granules contained within a basket, or similar assembly. The main innovations are related to (i) how the perfusion through the scaffolds is applied (not through any type of pump), and (ii) the combination of the features of a spinner flask with those of a direct perfusion system. The bioreactor thus represents a single closed system allowing combinations of the following processes: (i) cell expansion in suspension cultures; (ii) cell seeding into porous scaffolds by direct perfusion of the cell suspension; and (iii) cell culture into 3D scaffolds under perfusion.

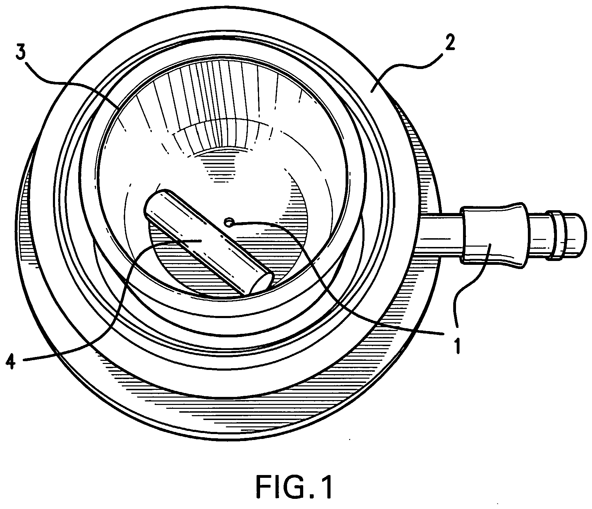

FIG. 1 shows an exemplary bottom unit, or end cap, of the present invention, consisting of a circular vessel with a flat bottom, containing a stirrer bar. Culture medium and cell suspensions may be filled and changed through an inlet/outlet port at the base of the chamber, fitted with a luer lock connector. The chamber is placed on a magnetic stirring plate, allowing stirring velocities down to 20 rpm. The reference characters are the following: (1) inlet/outlet luer lock port with opening at the center or the bottom; (2) bellows; (3) circular vessel (here made of PTFE, to minimize cell adhesion); and (4) magnetic stirring bar.

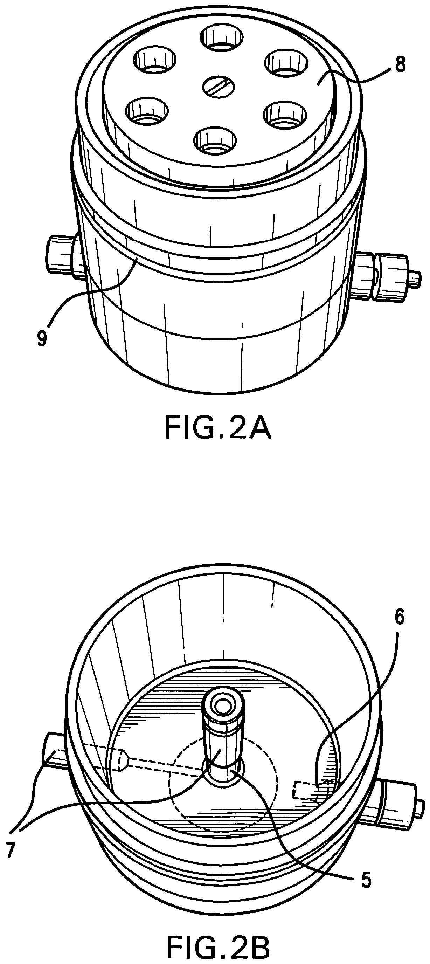

FIG. 2 shows an exemplary top unit, or end cap, of the present invention, consisting of a circular lid, placed centrally over the bottom unit and loosely in contact with the external side of the bottom circular vessel. In the center of the lid, there is a small hollow rod with a circular punch on the lower end. A punch serves as a scaffold holder and fits exactly the inner diameter of the bottom vessel. Fig. 2A shows the top unit with a scaffold holder and Fig. 2B shows the unit without a scaffold holder. The lid and punch can be moved up and down, from the upper border to the bottom of the lower vessel, with the punch stopping just above the stirring bar. Two inlet/outlet ports (or gas ports, as they are also suitable for use with gas of a controlled composition) with luer lock connectors allow air exchange through bacterial filters and culture medium filling or exchange through the hollow rod, with openings just above the scaffold holder.

The reference characters of FIG. 2 are as follows: (5) hollow rod; (6) inlet/outlet luer lock connector for air exchange; (7) inlet /outlet luer lock connector with opening just above the scaffold holder (punch) for medium exchange; (8) circular punch, fixed at the lower end of the rod with the function of a scaffold holder (this example for six scaffolds with a size of 8 x 5 mm); and (9) circular notch to insert the bellows for air tight closure after assembling with the bottom unit.

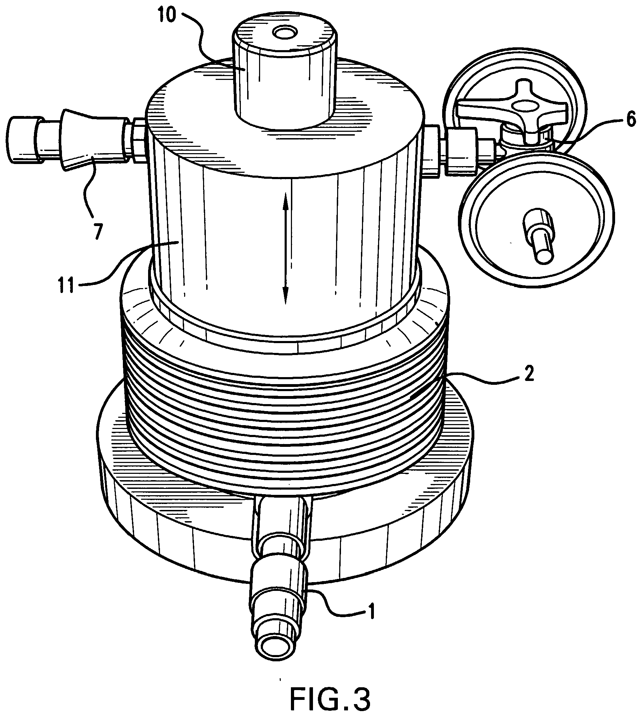

The top- and bottom units are assembled following mounting of the scaffolds and sterilization. An external airtight closure is achieved with an elastic bellows, thus ensuring free movement of the lid and sterility inside the bioreactor. FIG. 3 shows an exemplary assembled tissue culture unit. The reference characters are as follows: (1) bottom inlet/outlet with luer lock connector; (2) elastic bellows for an air-tight connection of the bottom- and top units; (6) air inlet/outlet, connected to bacterial filters with 0.2 µm pore size to ensure sterility inside the bioreactor; (10) connector to the linear driver motor; (7) inlet/outlet luer lock connector for medium exchange through the hollow rod in the center of the lid with an orifice just above the scaffold holder; and (11) top unit, movable up and down.

The motion of the top part of the bioreactor is performed by a linear actuator or a linear positioning table, driven by a stepper motor. Alternately, a rotary motor may be used when connected so as to produce a reciprocating motion. Other driving means may be used, for example a pneumatic or hydraulic drive. A control unit facilitates the application of different movement regimes (e.g., frequency, amplitude, ramp, plateau, and waveform). The closed and internally sterile tissue culture unit can be fixed at the bottom to the motion unit, and the lid is connected to the linear drive. The complete assembly is placed on a magnetic stirrer in an incubator at defined temperature, humidity, CO₂ and O₂ content.

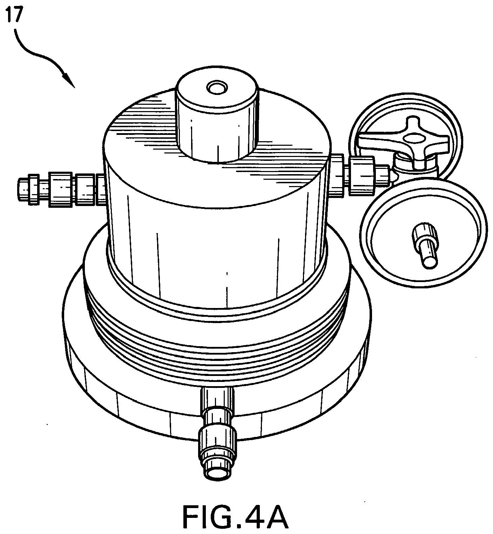

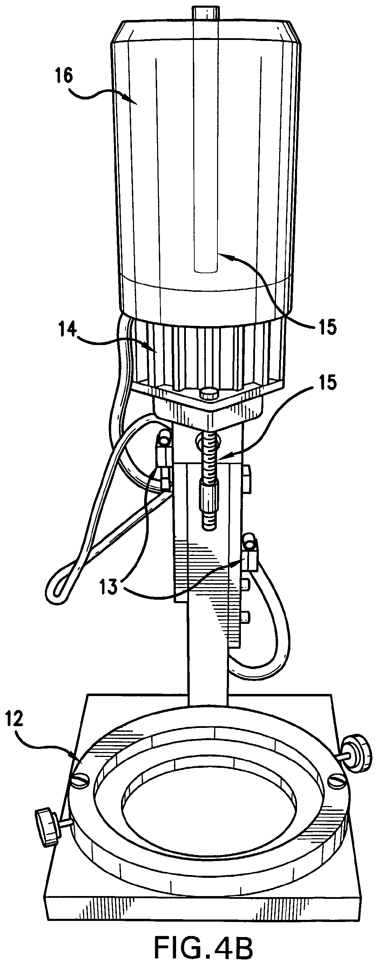

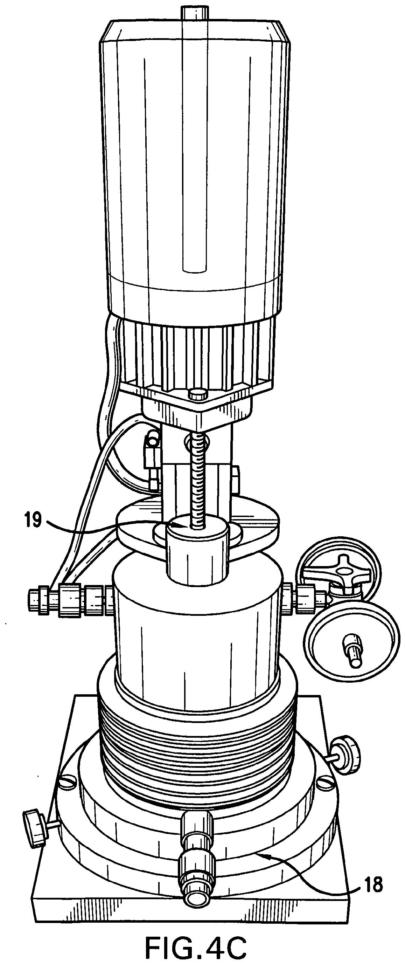

FIG. 4A shows an exemplary tissue culture unit of the present invention, FIG. 4B shows a exemplary linear drive unit according to the present invention, and FIG. 4C shows the tissue culture unit mounted to the drive unit. The reference characters are as follows: (12) holder with exact fit for bottom part of the tissue culture unit; (13) adjustable end- and start switches; (14) stepper motor; (15) threaded rod, disposed to move up and down, driven by the stepper motor; (16) motor cover to isolate the motor from condensed water and the humid atmosphere; (17) tissue culture unit; (18) bottom part of the tissue culture unit mounted and fixed in the holder of the motion unit; and (19) lid connected to the threaded rod, allowing linear movement of the top part of the tissue culture unit.

Scaffolds may be fabricated from a variety of biocompatible materials (e.g., synthetic polymers, natural polymers, metals, and ceramics) or tissue. Here a tissue refers to both living tissues, devitalized/decellularized tissue, reconstituted natural tissue, and equivalents. When the scaffold is of synthetic material, it should be a material suitable for cell growth, for example a foam, sponge, nonwoven mesh, gel, ceramic, or metal. When the scaffold comprises a tissue, including devitalized tissue, a large number of different tissues might be used, including for example dermis, bladder, bone, arteries, and heart valves

For a number of tissue types (e.g., liver, kidney, vascular) unidirectional flow is important for proper development and function. In the present invention this can be easily achieved by directly pumping medium through the reactor with the scaffold held motionless. This can be done following seeding of the scaffold with alternating perfusion.

The present invention may revitalize a tissue, or maintain viability of a living tissue. Such a tissue, according to the present invention, may then be implanted into a patient. The tissue may be allogenic or xenogenic. Clinically, the use of autologous cells is frequently preferred, however the cells need not be autologous.

### Exemplary methods of use

### Cell culture in suspension

During suspension culture, the punch (scaffold holder) of the bioreactor is positioned above the suspension culture with air in between the surface of the medium and the punch. The punch is moved up and down without touching the medium, thus ensuring air exchange (through bacterial filters) with the incubator environment. The cell suspension is stirred, avoiding cell settling and attachment to the walls, and furthermore inducing mixing of oxygen and nutrients throughout the medium. Medium can be changed through microfilters allowing flow of medium but not of cells. Culture in suspension has recently been shown to allow efficient expansion of bone marrow-derived stem cells (Baksh et al., Exp. Hematol. 31:723-732, 2003).

### Cell seeding into 3D-scaffolds

Following suspension culture for possible cell expansion, the lid with the punch (scaffold holder) can be moved down into the cell suspension, and then moved up and down from the medium surface to the bottom above the stirring stirrer bar. The cell suspension is thereby forced_{[z2]} through the pores of three dimensional scaffolds (direct perfusion) and the cells can adhere homogeneously to_{[z3]} the scaffold surface. Different movement regimes can be applied (e.g., frequency, plateau, amplitude, ramp, and waveform) to ensure maximum seeding efficiency and uniform cell distribution throughout the scaffold. The cell suspension can be continuously stirred during seeding, avoiding cell settling and assuring oxygen- and nutrient mixing. In one configuration, the whole punch can be replaced by a porous scaffold, thereby maximizing the volume of the cell-seeded material. Direct perfusion of a cell suspension through porous scaffolds has recently been shown to enhance seeding efficiency, uniformity, and the viability of the seeded cells (Wendt et al., Biotech Bioeng 84:205-214, 2003).

### Cell perfusion culture within scaffolds

Following cell seeding, subsequent culturing of the constructs is performed by the same oscillating movement of the scaffolds through the medium (now devoid of cells) as in phase 2. Dependent on the scaffold architecture and tissue to be generated, the velocity and motion regime of the punch needs to be defined to reach a careful balance between mass transfer of nutrients and cellular waste products, retention of newly synthesized extracellular matrix components within the constructs, and fluid induced shear stresses within the pores. Medium changes can be performed through the available two ports (connectors) either at specific time points or continuously with an external pump. Optional additional stirring of the medium during perfusion cultures can be performed to improve mass transfer.

The system can be used to perform some or all of the three above-described culture phases. Performing the three culture phases is foreseen to be employed to generate bone-like constructs starting from bone marrow-derived cells expanded in suspension, and later seeded and cultured in/on porous scaffolds. The bone-like constructs can be used as osteoinductive grafts or as a system to expand hematopoietic stem cells within a 3D stromal tissue.

While the present invention has been described with reference to certain preferred embodiments, one of ordinary skill in the art will recognize that additions, deletions, substitutions, modifications and improvements can be made.

The figures refer to the culture chamber as approximately cylindrical with approximately circular caps, however the end caps of the present invention need not be circular caps and can take other shapes depending on the shape of the culture chamber. Although removable end caps are preferred for ease of access to the culture chamber, the chamber of the present invention is not limited to one with removable caps.

Although the examples discuss an embodiment wherein the scaffold moves in conjunction with an end cap, the invention may also be performed by causing relative motion between the scaffold and at least one end cap, without necessarily having motion of the end caps relative to each other.

## Claims

1. A method of growing cells in culture, comprising the steps of:
(a) providing a three-dimensional porous scaffold disposed in a culture chamber, the culture chamber comprising a space between a first end cap and a second end cap;
(b) providing one or more living cells;
(c) providing a liquid growth medium; and
(d) causing relative motion between the scaffold and at least one end cap, thereby moving the growth medium through the scaffold,
**characterized in that**,
a holder configured to hold the porous, three-dimensional scaffold for growing or maintaining cells is integral with the one said end cap,
a hollow rod is, at one end, attached to the center of said one of said end caps and, at the other end, to a punch serving as said holder and fitting an interior diameter of the culture chamber, and,
during step (d), said scaffold moves in conjunction with the one said end cap.

2. The method of claim 1, wherein said scaffold may alternately be disposed in one of (1) an position out of said growth medium, or (2) a lower position in said growth medium; the method further comprising the steps, before step (d), of:
growing said cells in a suspension in said growth medium while said scaffold is disposed in the upper position, and
lowering said scaffold into the lower position.

3. The method of claim 1, wherein said culture chamber further comprises connectors for flowing said growth medium in and out of said culture chamber.

4. The method of claim 1, wherein said culture chamber further comprises a stirrer bar.

5. The method of claim 1, further comprising a step, before step (d), of growing said cells in a suspension in said growth medium.

6. A bioreactor for growing cells in culture, comprising:
(a) a culture chamber including a first end cap and a second end cap connected to define a space; and
(b) a holder configured to hold a porous, three-dimensional scaffold for growing or maintaining cells;
wherein the holder is disposed between the end caps to allow a reciprocating motion between the holder and at least one of said end caps, **characterized in that**
said holder is integral with one of said end caps and
a hollow rod is, at one end, attached to the center of said one of said end caps and, at the other end, to a punch serving as said holder and fitting an interior diameter of the culture chamber.

7. The bioreactor of claim 6, wherein said holder moves in conjunction with one said end cap.

8. The bioreactor of claim 6, further comprising a drive means operationally connected to actuate said reciprocating motion.

9. The bioreactor of claim 6, wherein the holder may alternately be disposed in one of (1) an upper position to dispose a held scaffold out of a growth medium, or (2) a lower position to dispose a held scaffold in a growth medium.

10. The bioreactor of claim 6, wherein said culture chamber further comprises connections disposed to permit flow of a growth medium in and out of said culture chamber.

11. The bioreactor of claim 6, further comprising a gas port connected to said culture chamber.

12. The bioreactor of claim 6, further comprising a stirring unit for growing cells in suspension.

13. The bioreactor of claim 6, further comprising a porous, three-dimensional scaffold for growing or maintaining cells.

14. The method of any of claims 1-5, wherein said scaffold comprises a tissue.

15. The bioreactor of any of claims 6-13, wherein said scaffold comprises a tissue.

16. The method of claim 1, further comprising causing unidirectional perfusion of said medium through said scaffold.

17. The bioreactor of claim 6, further comprising two ports for a growth media disposed so that, when said bioreactor houses said scaffold, the growth media is flowable through the ports and said scaffold in a unidirectional manner.

## Patentansprüche

1. Verfahren zum Wachsen von Zellen in Kultur, umfassend die Schritte:
(a) Bereitstellen eines dreidimensionalen porösen Scaffolds, das in einer Kulturkammer angeordnet ist, wobei die Kulturkammer einen Raum zwischen einer ersten Endkappe und einer zweiten Endkappe umfasst;
(b) Bereitstellen einer oder mehrerer lebender Zellen;
(c) Bereitstellen eines flüssigen Wachstumsmediums; und
(d) Bewirken einer relativen Bewegung zwischen dem Scaffold und mindestens einer Endkappe, wodurch das Wachstumsmedium durch das Scaffold hindurch bewegt wird,
**dadurch gekennzeichnet, dass**
ein Halter, der zum Halten des porösen dreidimensionalen Scaffolds zum Wachsen oder Erhalten von Zellen ausgestaltet ist, einstückig mit der einen Endkappe ist,
eine hohle Stange an einem Ende an der Mitte der einen der Endkappen und am anderen Ende an einem Stempel befestigt ist, der als der Halter dient und in einen Innendurchmesser der Kulturkammer passt, und
sich das Scaffold während des Schritts (d) zusammen mit der einen Endkappe bewegt.

2. Verfahren nach Anspruch 1, wobei das Scaffold alternativ in einer von (1) einer Position außerhalb des Wachstumsmediums oder (2) einer unteren Position in dem Wachstumsmedium angeordnet sein kann; wobei das Verfahren vor Schritt (d) ferner die Schritte umfasst:
Wachsen der Zellen in einer Suspension in dem Wachstumsmedium, während das Scaffold in der oberen Position angeordnet ist, und
Absenken des Scaffolds in die untere Position.

3. Verfahren nach Anspruch 1, wobei die Kulturkammer ferner Verbinder zum Strömenlassen des Wachstumsmediums in die und aus der Kulturkammer umfasst.

4. Verfahren nach Anspruch 1, wobei die Kulturkammer ferner einen Rührstab umfasst.

5. Verfahren nach Anspruch 1, das ferner vor Schritt (d) einen Schritt des Wachsens der Zellen in einer Suspension in dem Wachstumsmedium umfasst.

6. Bioreaktor zum Wachsen von Zellen in Kultur, umfassend:
(a) eine Kulturkammer mit einer ersten Endkappe und einer zweiten Endkappe, die verbunden sind, um einen Raum zu definieren; und
(b) einen Halter, der zum Halten eines porösen dreidimensionalen Scaffolds zum Wachsen oder Erhalten von Zellen ausgestaltet ist;
wobei der Halter zwischen den Endkappen angeordnet ist, um eine Hin- und Herbewegung zwischen dem Halter und mindestens einer der Endkappen zu ermöglichen, **dadurch gekennzeichnet, dass**
der Halter einstückig mit einer der Endkappen ist und eine hohle Stange an einem Ende an der Mitte der einen der Endkappen und am anderen Ende an einem Stempel, der als der Halter dient und in einen Innendurchmesser der Kulturkammer passt, befestigt ist.

7. Bioreaktor nach Anspruch 6, wobei sich der Halter zusammen mit der einen Endkappe bewegt.

8. Bioreaktor nach Anspruch 6, ferner umfassend ein Antriebsmittel, das operational zum Antreiben der Hin- und Herbewegung verbunden ist.

9. Bioreaktor nach Anspruch 6, wobei der Halter alternativ in einer von (1) einer oberen Position, um ein gehaltenes Scaffold aus einem Wachstumsmedium zu entnehmen, oder (2) einer unteren Position, um ein gehaltenes Scaffold in einem Wachstumsmedium anzuordnen, angeordnet sein kann.

10. Bioreaktor nach Anspruch 6, wobei die Kulturkammer ferner Verbindungen aufweist, die so angeordnet sind, dass ein Strömen eines Wachstumsmediums in die Kulturkammer und aus dieser heraus ermöglicht ist.

11. Bioreaktor nach Anspruch 6, ferner umfassend einen Gasanschluss, der mit der Kulturkammer verbunden ist.

12. Bioreaktor nach Anspruch 6, ferner umfassend eine Rühreinheit zum Wachsen von Zellen in Suspension.

13. Bioreaktor nach Anspruch 6, ferner umfassend ein poröses dreidimensionales Scaffold zum Wachsen oder Erhalten von Zellen.

14. Verfahren nach einem der Ansprüche 1-5, wobei das Scaffold ein Gewebe umfasst.

15. Bioreaktor nach einem der Ansprüche 6-13, wobei das Scaffold ein Gewebe umfasst.

16. Verfahren nach Anspruch 1, ferner umfassend das Bewirken einer unidirektionalen Perfusion des Mediums durch das Scaffold.

17. Bioreaktor nach Anspruch 6, ferner umfassend zwei Anschlüsse für ein Wachstumsmedium, die derart angeordnet sind, dass, wenn der Bioreaktor das Scaffold beherbergt, das Wachstumsmedium auf unidirektionale Weise durch die Anschlüsse und das Scaffold strömen kann.

## Revendications

1. Procédé de croissance de cellules en culture, comprenant les étapes consistant à:
(a) fournir un échafaudage tridimensionnel poreux disposé dans une chambre de culture, la chambre de culture comprenant un espace entre un premier capuchon d'extrémité et un second capuchon d'extrémité;
(b) fournir une ou plusieurs cellules vivantes;
(c) fournir un milieu de croissance liquide; et
(d) provoquer un mouvement relatif entre l'échafaudage et au moins un capuchon d'extrémité, déplaçant ainsi le milieu de croissance à travers l'échafaudage,
**caractérisé en ce que**
un support conçu pour supporter l'échafaudage tridimensionnel poreux pour la croissance ou le maintien de cellules solidaire de l'un desdits capuchons d'extrémité,
une tige creuse est fixée, à une extrémité, au centre dudit capuchon d'extrémité et, à l'autre extrémité, à un poinçon servant audit support et s'adaptant à un diamètre intérieur de la chambre de culture et
au cours de l'étape (d), ledit échafaudage se déplace conjointement avec ledit un capuchon d'extrémité.

2. Procédé selon la revendication 1, dans lequel ledit échafaudage peut alternativement être disposé dans (1) une position hors dudit milieu de croissance ou (2) une position inférieure dans ledit milieu de croissance; le procédé comprenant en outre, avant l'étape (d), les étapes consistant à:
faire croître lesdites cellules dans une suspension dans ledit milieu de croissance pendant que ledit échafaudage est disposé dans la position supérieure et abaisser ledit échafaudage dans la position inférieure.

3. Procédé selon la revendication 1, dans lequel ladite chambre de culture comprend en outre des raccords pour faire pénétrer ledit milieu de croissance dans ladite chambre de culture et l'en évacuer.

4. Procédé selon la revendication 1, dans lequel ladite chambre de culture comprend en outre un barreau agitateur.

5. Procédé selon la revendication 1, comprenant en outre une étape, avant l'étape (d), de croissance desdites cellules dans une suspension dans ledit milieu de croissance.

6. Bioréacteur pour la croissance de cellules en culture, comprenant:
(a) une chambre de culture comprenant un premier capuchon d'extrémité et un second capuchon d'extrémité reliés pour définir un espace; et
(b) un support conçu pour supporter un échafaudage tridimensionnel poreux pour la croissance ou le maintien de cellules;
dans lequel le support est disposé entre les capuchons d'extrémité pour permettre un mouvement de va-et-vient entre le support et au moins l'un desdits capuchons d'extrémité, **caractérisé en ce que**
ledit support est solidaire de l'un desdits capuchons d'extrémité et une tige creuse est, à une extrémité, fixée au centre dudit capuchon d'extrémité et, à l'autre extrémité, à un poinçon servant audit support et s'adaptant à un diamètre intérieur de la chambre de culture.

7. Bioréacteur selon la revendication 6, dans lequel ledit support se déplace conjointement avec ledit un desdits capuchons d'extrémité.

8. Bioréacteur selon la revendication 6, comprenant en outre un moyen d'entraînement relié de manière opérationnelle pour activer ledit mouvement de va-et-vient.

9. Bioréacteur selon la revendication 6, dans lequel le support peut alternativement être disposé dans (1) une position supérieure pour disposer hors d'un milieu de croissance un échafaudage maintenu ou (2) une position inférieure pour disposer un échafaudage maintenu dans un milieu de croissance.

10. Bioréacteur selon la revendication 6, dans lequel ladite chambre de culture comprend en outre des raccordements disposés pour permettre à un milieu de croissance de pénétrer dans ladite chambre de culture et d'en être évacué.

11. Bioréacteur selon la revendication 6, comprenant en outre un orifice de gaz relié à ladite chambre de culture.

12. Bioréacteur selon la revendication 6, comprenant en outre une unité d'agitation pour la croissance de cellules en suspension.

13. Bioréacteur selon la revendication 6, comprenant en outre un échafaudage tridimensionnel poreux pour la croissance ou le maintien de cellules.

14. Procédé selon l'une quelconque des revendications 1 à 5, dans lequel ledit échafaudage comprend un tissu.

15. Bioréacteur selon l'une quelconque des revendications 6 à 13, dans lequel ledit échafaudage comprend un tissu.

16. Procédé selon la revendication 1, comprenant en outre l'étape consistant à provoquer une perfusion unidirectionnelle dudit milieu à travers ledit échafaudage.

17. Bioréacteur selon la revendication 6, comprenant en outre deux orifices pour un milieu de croissance disposés de sorte que, lorsque ledit bioréacteur loge ledit échafaudage, le milieu de croissance puisse passer par les orifices et ledit échafaudage de manière unidirectionnelle.
